# EUROPEAN PATENT SPECIFICATION

(11) **EP 0 882 136 B1**
(45) Date of publication and mention of the grant of the patent: **17.04.2002**
(21) Application number: 97904044.1
(22) Date of filing: 31.01.1997
(51) Int. Cl.: C12P 7/62, C12N 9/16, A61K 47/14

(54) **METHODS FOR MAKING LYSOPHOSPHATIDYLCHOLINE**
VERFAHREN ZUR HERSTELLUNG VON LYSOPHOSPHATIDYLCHOLIN
PROCEDES DE PREPARATION DE LYSOPHOSPHATIDYLCHOLINE

(30) Priority: 02.02.1996 US 597450
(43) Date of publication of application: 09.12.1998
(73) Proprietor: Biomolecular Products, Inc., Byfield, MA 01922 (US)
(72) Inventor: YESAIR, David, W., Byfield, MA 01922 (US)
(74) Representative: Jump, Timothy John Simon
(86) International application number: PCT/US97/01367
(87) International publication number: WO 97/28270

(56) References cited:
- WO-A-93/13219
- KR-B- 9 503 530
- US-A- 4 874 795
- DATABASE WPI Section Ch, Week 9342 Derwent Publications Ltd., London, GB; Class B05, AN 93-330587 XP002033363 & JP 05 236 974 A (NICHIRO KK) , 17 September 1993
- DATABASE WPI Section Ch, Week 9013 Derwent Publications Ltd., London, GB; Class D16, AN 90-096521 XP002033364 & JP 02 049 593 A (SHOWA SANGYO CO) , 19 February 1990
- DATABASE WPI Section Ch, Week 8847 Derwent Publications Ltd., London, GB; Class D13, AN 88-334701 XP002033365 & JP 63 248 430 A (NISSHIN OIL MILLS LTD) , 14 October 1988
- DATABASE WPI Section Ch, Week 8901 Derwent Publications Ltd., London, GB; Class D13, AN 89-003045 XP002033366 & JP 63 279 753 A (TAIYO CHEM IND CO LTD) , 16 November 1988
- DATABASE WPI Section Ch, Week 9108 Derwent Publications Ltd., London, GB; Class B05, AN 91-055757 XP002033367 & JP 03 007 589 A (NIPPON OILS & FATS CO LTD) , 14 January 1991
- DATABASE WPI Section Ch, Week 8943 Derwent Publications Ltd., London, GB; Class D23, AN 89-314148 XP002033368 & JP 01 233 290 A (NIPPON OILS & FATS CO LTD) , 19 September 1989

## Description

### Field of the Invention

This invention relates generally to the field of phospholipid hydrolysis. In particular, this invention relates to an improved method of phospholipase A₂ hydrolysis of phosphatidylcholine to produce lysophosphatidylcholine. This invention also relates to a method of making a lipid matrix comprising lysophosphatidylcholine, monoglyceride, and fatty acid.

### Background of the Invention

Enzymatic conversion of phosphatidylcholine to lysophosphatidylcholine has been known since the early 1900's. Early investigations of the degradation of lecithin (phosphatidylcholine) by snake venom extracts demonstrated that the action of snake venom hemolysis is upon the lecithin portion of the cell membrane. In 1935, Hughes demonstrated that the hydrolysis of a unimolecular film of lecithin to lysolecithin (lysophosphatidylcholine) is dependent on factors such as pH, temperature and the surface concentration of the lecithin molecules. Packing of the lecithin molecules in the unimolecular layer greatly decreased the rate of hydrolysis. Hanahan demonstrated that an ether-soluble complex between egg phosphatidylcholine and phospholipase A₂ resulted in the release of unsaturated fatty acid and lysophosphatidylcholine. Hydrolysis of phosphatidylcholine by phospholipase A₂ could not be detected when 95% ethyl alcohol, chloroform or petroleum ether were used as solvents. Experiments performed by Dawson, reported in 1963, also found that phospholipase A₂ hydrolyzed phosphatidylcholine to lysophosphatidylcholine and a single fatty acid molecule. Dawson determined that the enzymatic activity was dependent on the presence of calcium ions, and that the addition of ether or butanol stimulated the phospholipase A₂ activity. British patent 1,215,868 to Unilever Ltd. described a further modification of the hydrolysis of phospholipid by phospholipase A₂, conducting the reaction in the presence of fat (oils).

The processes of phosphatidylcholine hydrolysis disclosed in the prior art suffer from several shortcomings, including incomplete hydrolysis and production of unwanted side products in the hydrolysis reaction. The deficiencies of the prior art methods are severe because the presence of unreacted starting materials or unwanted side products represent an unacceptable level of contaminants in the final reaction product. These unwanted constituents must be removed from the reaction product in order to obtain the desired product, lysophosphatidylcholine, thus necessitating additional purification steps.

The prior art methods described above produce a maximal yield of lysophosphatidylcholine of approximately 70% of the starting phosphatidylcholine. Dawson showed that the addition of ether was required to stimulate the phospholipase A₂ activity in the hydrolysis of phosphatidylcholine to the maximum yield of about 60-70%. The maximum yield of lysophosphatidylcholine was obtained when 8% diethyl ether (vol./vol.) in aqueous buffer was the reaction medium; using this reaction medium a two-phase system was observed. Dawson also found that 6% butanol (vol./vol.) could substitute for diethyl ether in the reaction medium to enhance yield of lysophosphatidylcholine, but ethanol and methylisobutylhexane were ineffective for increasing hydrolysis of phosphatidylcholine. Dawson concluded that the stimulatory effect of ether (or butanol) on hydrolysis of phosphatidylcholine was probably due to surface dilution of the closely packed phosphatidylcholine molecules oriented at the lipid interface and a removal of inhibitory fatty acid carbonyl groups from the interface. This conclusion was supported by evidence that addition of fatty acids inhibited the enzymatic hydrolysis of phosphatidylcholine (Dawson). Inhibition of the reaction by added fatty acid resulted either from inhibiting the removal of the fatty acid from the interface, or from formation of a calcium ion - fatty acid chelate, i.e., removal of Ca²⁺ ions required for phospholipase A₂ activity. Dawson believed that the removal of calcium ions was the more likely explanation because the further addition of ether to form two phases and solubilize the additional fatty acid did not promote hydrolysis of phosphatidylcholine, whereas increasing the calcium concentration ten fold did partially relieve the inhibition. It was also shown that the phospholipase A₂ enzyme purified from cobra venom was dependent on the presence of calcium ions for hydrolysis activity. The requirement for calcium ions in the hydrolysis reaction by phospholipase A₂ and the association of calcium ions with fatty acids released by the hydrolysis of phosphatidylcholine is well known in the art (Novo Nordisk).

The present inventor has previously invented methods for the preparation of mixed lipid particles useful in the delivery of drugs and for providing readily absorbable calories to an individual (U.S. patents no. 4,874,795 and 5,314,921). These methods involve the mixing of iysophosphatidylcholine, monoglyceride and fatty acid in specific molar ratios. Although easily performed, these previous methods use costly, isolated, highly purified lysophosphatidylcholine, thus adding to the expense of the final mixed lipid particle product.

Because of the deficiencies of the prior art noted above, at the present time there is a need for methods by which phosphatidylcholine is more efficiently converted to lysophosphatidylcholine. Such a process would result in more efficient use of phosphatidylcholine and yield fewer unwanted side products (such as glycerophosphatidylcholine) and contaminants (such as unhydrolyzed phosphatidylcholine)of the final reaction product. The use of a method in which the end products are in a more pure form would result in substantial cost savings and time saving due to a reduced need for the purification of the end products. There is also a need for a simplified method of producing mixed lipid particles comprising lysophosphatidylcholine, monoglyceride and fatty acid. A method which utilizes phosphatidylcholine as a starting material would reduce the need for the use of a purified lysophosphatidylcholine as a starting material, thus reducing the overall costs for the final mixed lipid particle product.

### Summary of the Invention

It is an object of the invention to provide a method for making lysophosphatidylcholine which is more efficient than prior art methods. For example, prior art methods of making lysophosphatidylcholine typically achieve no better than 60-70% efficient conversion of phosphatidylcholine starting material. The present invention provides in preferred embodiments a method whereby phosphatidylcholine may be converted to lysophosphatidylcholine with nearly 100% efficiency. Further, the hydrolysis of phosphatidylcholine according to the present invention results in production of small quantities, if any, of unwanted side products. The present invention provides a method which reduces the cost of making lysophosphatidylcholine by converting phosphatidylcholine with nearly 100% efficiency.

The invention involves improvements in enzymatic hydrolysis of phosphatidylcholine. According to the invention, combining certain agents with phosphatidylcholine increases the efficiency of enzymatic hydrolysis of phosphatidylcholine to lysophosphatidylcholine and fatty acid. relative to hydrolysis of phosphatidylcholine without addition of such agents. Thus, the invention provides improved methods for making lysophosphatidylcholine. It also provides improved methods for making compositions containing lysophosphatidylcholine, monoglyceride and fatty acid. The improved methods include enzymatic hydrolysis of an aqueous dispersion of phosphatidylcholine and an agent which enhances the hydrolysis of phosphatidylcholine. To provide biologically preferred forms of lysophosphatidylcholine, the enzyme which hydrolyzes phosphatidylcholine preferably removes a fatty acid from the 2-position of phosphatidylcholine, such as phospholipase A₂.

According to one aspect of the invention, methods for making lysophosphatidylcholine are provided. An agent and phosphatidylcholine are combined with water to form in the water an aqueous dispersion of a mixture of the agent and phosphatidylcholine. The aqueous dispersion of the mixture is contacted with phospholipase A₂ to form a reaction mixture, preferably in the presence of calcium ions. The agent can be monoglyceride, diglyceride, polyglycerol fatty acid ester, sucrose fatty acid ester, sorbitan fatty acid ester or glycerol. In certain embodiments, the phosphatidylcholine in the mixture is less than about 40% by weight of the mixture, and preferably is about 30% by weight of the mixture. In certain embodiments, the method further includes recovering the lysophosphatidylcholine formed in the reaction mixture. Preferably, recovering the lysophosphatidylcholine includes separating the lysophosphatidylcholine from a reaction mixture constituent selected from the group consisting of fatty acid, the agent, or fatty acid and the agent. In a particularly preferred embodiment, the latter separation includes extraction with acetone. The invention thus provides a method for separation of lysophosphatidylcholine from all of the aforementioned components, such that the resulting substantially pure product may be used without further purification. In any of the foregoing embodiments, the agent preferably is monoglyceride, most preferably a monoglyceride which has an acyl group having between 8 and 22 carbon atoms.

According to another aspect of the invention, methods for making lysophosphatidylcholine are provided. An agent, phosphatidylcholine and an organic solvent are combined with water to form in the water an aqueous dispersion, containing the organic solvent, of a mixture of the agent and phosphatidylcholine. The aqueous dispersion of the mixture is contacted with phospholipase A₂ to form a reaction mixture, preferably in the presence of calcium ions. The agent can be monoglyceride, diglyceride, polyglycerol fatty acid ester, sucrose fatty acid ester, sorbitan fatty acid ester or glycerol. In preferred embodiments, the organic solvent is tertiary butyl alcohol. In additional embodiments, the organic solvent may be diethyl ether, a mixture of diethyl ether and ethanol, preferably in which the ethanol represents about 4% of the total organic solvent, a mixture of tertiary butyl alcohol and ethanol, preferably in which the ethanol represents about 4% of the total organic solvent, or methyl isobutyl ketone.

According to still another aspect of the invention, methods of making lysophosphatidylcholine are provided in which an agent and phosphatidylcholine are combined with water to form in the water an aqueous dispersion. The aqueous dispersion is contacted with phospholipase A₂ to form a reaction mixture, wherein the agent is present in an amount effective to enhance the conversion of phosphatidylcholine to lysophosphatidylcholine in the reaction mixture compared to the conversion of phosphatidylcholine to lysophosphatidylcholine in the reaction mixture absent the agent. Preferably, the agent is present in an amount effective to increase the efficiency of the reaction to 80% or greater, more preferably 90% or greater, even more preferably 95% or greater, or most preferably 99% or greater. The agent preferably is an agent selected from the group consisting of monoglyceride, diglyceride, polyglycerol fatty acid ester, sucrose fatty acid ester, sorbitan fatty acid ester or glycerol.

While virtually any amount of the preferred agent, monoglyceride, will enhance hydrolysis of phosphatidylcholine by phospholipase A₂, according to another aspect of the invention methods of making lysophosphatidylcholine are provided in which the molar ratio of phosphatidylcholine:monoglyceride is from about 1:1 to about 1:5. Preferably, the molar ratio of phosphatidylcholine to monoglyceride is about 1:3. According to these embodiments of the invention, hydrolysis of phosphatidylcholine proceeds with nearly 100% efficiency.

Virtually any monoglyceride is useful for enhancing the hydrolysis of phosphatidylcholine by phospholipase A₂. However, according to preferred embodiments, the monoglyceride useful in the invention may have an acyl group consisting of anywhere between 8 and 22 carbon atoms. It is preferred that the acyl group of the monoglyceride has between 1 and 4 unsaturations, i.e., double carbon-carbon bonds. Thus, it is preferred that the monoglyceride in the mixture comprises an acyl group selected from the group consisting of an acyl group having one unsaturation, an acyl group having two unsaturations, an acyl group having three unsaturations and an acyl group having four unsaturations. Most preferably, more than 50% of the monoglyceride in the mixture comprises an acyl group selected from the aforementioned group of acyl groups having one, two, three or four unsaturations. Thus, according to the invention, specific monoglycerides may be selected based on the ability of the monoglyceride molecules to separate the phosphatidylcholine molecules for efficient hydrolysis of phosphatidylcholine, and further based on the length and unsaturation of the acyl group which may be desired in useful end products of the reaction, such as lipid matrix compositions described below.

According to another aspect of the invention, methods for making a lipid matrix composition containing lysophosphatidylcholine, monoglyceride and fatty acid are provided. Phosphatidylcholine and monoglyceride are contacted with the phospholipase A₂, and the resultant lipid complex is recovered, for example by the removal of water. If desired, monoglyceride and/or fatty acid may be added to the lipid complex to obtain a preferred molar ratio of the lipid complex components. According to a preferred embodiment, the lipid complex may have a molar ratio of lysophosphatidylcholine:the sum of monoglyceride and fatty acid of between about 1:4 and 1:12. More preferably, the molar ratio of lysophosphatidylcholine:the sum of monoglyceride and fatty acid may be between about 1:5 and 1:6. In another preferred embodiment, the constituents of the lipid complex may be present in a molar ratio of lysophosphatidylcholine:monoglyceride:fatty acid of between 1:4:2 and 1:2:4. Most preferably, the lipid complex may consist of lysophosphatidylcholine, monoglyceride and fatty acid in a molar ratio of lysophosphatidylcholine:monoglyceride:fatty acid of :4:2, 1:3:3 or 1:3:2. In an additional embodiment, the method of making a lipid matrix containing lysophosphatidylcholine, monoglyceride and fatty acid may include the use of a monoglyceride which is derived from natural triglyceride.

According to yet another aspect of the invention, methods for making lysophosphatidylcholine are provided in which an agent and phosphatidylcholine are combined to form a mixture of the agent and phosphatidylcholine. The agent can be monoglyceride, diglyceride, polyglycerol fatty acid ester, sucrose fatty acid ester, sorbitan fatty acid ester or glycerol. The mixture is combined with water to form in the water an aqueous dispersion of the mixture which dispersion is contacted with phospholipase A₂ to form a reaction mixture, preferably in the presence of calcium ions. The lysophosphatidylcholine then can be recovered.

Although not wishing to be bound by any theory of the invention, it is believed that, according to the methods of the invention, the lamellar structure of phosphatidylcholine/lysophosphatidylcholine/water as described by Small (J. Am. Oil Chemists' Soc., 45:108-119. 1968) is maintained during the sequential enzymatic hydrolysis of phosphatidylcholine/agent/water to lysophosphatidylcholine/agent/fatty acid/water. By providing a set of reaction conditions under which the lamellar structure is maintained, the efficiency of the hydrolysis reaction is enhanced because phosphatidylcholine molecules remain accessible to the phospholipase A₂ enzyme throughout the course of the reaction.

These and other aspects and objects of the invention will be described in further detail in connection with the Detailed Description of the Invention.

### Brief Description of the Figures

Figure 1 depicts the composition over time of the phosphatidylcholine hydrolysis reaction mixture without added monoolein.

Figure 2 depicts the composition over time of the phosphatidylcholine hydrolysis reaction mixture with monoolein added at a 1:3 molar ratio.

### Detailed Description of the Invention

The invention is an improved method for making lysophosphatidylcholine which involves contacting phosphatidylcholine and an agent with phospholipase A₂. Phospholipase A₂ is preferred because the lysophosphatidylcholine produced by hydrolysis of phosphatidylcholine at the 2-position is the biologically preferred lysophosphatidylcholine (in contrast with lysophosphatidylcholine produced by hydrolysis of phosphatidylcholine at the 1-position). The formed lysophosphatidylcholine optionally may be separated from the added agent and/or the fatty acids which are liberated by the action of phospholipase A₂ on phosphatidylcholine. The method enables substantially complete hydrolysis of phosphatidylcholine to lysophosphatidylcholine in a single step. If desired, the agent may be monoglyceride and the resulting lipid matrix of lysophosphatidylcholine, monoglyceride and fatty acids may be separated from the phospholipase A₂, trace unreacted phosphatidylcholine, water, organic solvents, and any impurities present in the reaction mixture.

The starting material for the method is phosphatidylcholine, a phospholipid composed of a polar hydrophilic head group of choline, phosphate and glycerol linked to a nonpolar hydrophobic tail group consisting of two fatty acid molecules. Phosphatidylcholine may be obtained with specific fatty acid groups, or with a mixture of various fatty acid groups. For example, Phospholipon® 80 (American Lecithin, Oxford, CT), is a mixture of phosphatidylcholine molecules having a variety of fatty acid acyl groups linked to the polar head group.

As disclosed herein, a mixture of phosphatidylcholine and an agent and optionally other reaction components is prepared by combining these reaction components. The term "mixture" merely indicates that the components are in contact with one another. For example, the mixture can be a colloidal mixture of phosphatidylcholine and agent; when combined with water or other aqueous solvents, it can be said that colloids of a mixture of phosphatidylcholine and the agent are dispersed in the water. The mixture also can be dispersed in the water not as small particles but as larger colloidal or noncolloidal complexes. It is believed that the phosphatidylcholine and the agent form bi- or multilayered structures, although the invention is not limited by this theory. Thus, mixtures of different physical form or structure are embraced by the invention, provided that the mixture enhances the activity of phospholipase A₂ in converting phosphatidylcholine to lysophosphatidylcholine.

In the methods of the present invention, an agent and phosphatidylcholine are combined with water to make an aqueous dispersion of the mixture of phosphatidylcholine and the agent, and the dispersion is subsequently contacted with phospholipase A₂ under conditions which permit hydrolysis of the phosphatidylcholine molecules. It is believed that phosphatidylcholine in water or virtually any aqueous solvent will form in the water or aqueous solvent a dispersion. In other words, "water" as used herein is meant to embrace water and other aqueous buffers compatible with forming a dispersion of the mixture of phosphatidylcholine and the agent. As mentioned above, it is believed that this dispersion will involve a lamellar (bilayer or multilayer) structure with the polar head groups of phosphatidylcholine oriented to the outside of the bilayer or multilayer. It further is believed that phosphatidylcholine when mixed with an agent of the invention and heated also will form a dispersion in the aqueous solvent and maintain a similar lamellar structure. The addition of an agent is believed to achieve several purposes. First, the molecules of the agent are believed to separate the phosphatidylcholine molecules to allow greater access to the phosphatidylcholine by phospholipase A₂, thus enabling complete hydrolysis to lysophosphatidylcholine. Second, addition of the agent is believed to maintain the lamellar structure during the hydrolysis reaction. Third, addition of the agent is believed to maintain fluidity of the phosphatidylcholine bilayer to enhance hydrolysis by phospholipase A₂. Thus, any agent which has one or more of the aforementioned characteristics is believed suitable for adding to phosphatidylcholine to facilitate hydrolysis by phospholipase A₂. It is preferable that the agent be selected from amongst the group consisting of monoglyceride, diglyceride, polyglycerol fatty acid ester, sucrose fatty acid ester, sorbitan fatty acid ester and glycerol. Most preferably, the agent is monoglyceride.

Monoglyceride is composed of a glycerol head group to which one fatty acid acyl group is attached. Preferred acyl groups of monoglyceride useful in the invention may range in number of carbon atoms from 8 to 22. Acyl groups of monoglyceride may be saturated or unsaturated, preferably with 1-4 double bonds in the carbon chain. The monoglyceride may be highly purified or may be added in a crude form, depending on the needs of the user and the tolerance for impurities in the reaction mixture. Monoglycerides useful in the invention may represent a mixture of monoglyceride molecules having different size and saturation-state acyl groups, or the monoglyceride may represent only a single type of acyl group, e.g., mono-olein, mono-palmitin. Examples of a mixture of monoglycerides useful in the invention include Dimodan™ LSK and Dimodan™ OK (Danisco Ingredients USA, Inc., New Century, KS).

Diglyceride molecules are also useful in the method of the invention for enhancing the hydrolysis of phosphatidylcholine by phospholipase A₂. A diglyceride molecule consists of a glycerol head group to which two fatty acid acyl groups are attached. As with the acyl group of monoglyceride, the acyl groups of diglyceride preferably have carbon chain links from 8 to 22 carbon atoms and 1 to 4 unsaturations. As with monoglyceride, the specific acyl groups, purity, and mixture of diglyceride molecules useful in the invention depend on the requirements of the individual user. Any combination or type of diglyceride molecules is contemplated by the invention, so long as the hydrolysis of phosphatidylcholine is enhanced.

Other agents such as polyglycerol fatty acid esters, sorbitan fatty acid esters, sucrose fatty acid esters and glycerol may also enhance the hydrolysis of phosphatidylcholine by separation of the phosphatidylcholine molecules and maintenance of a lamellar structure and fluidity. Such compounds are described in U.S. patent no. 4,849,132. A polyglycerol fatty acid ester molecule consists of mono-. di- or polyesters of fatty acids with 4-12 polymerized glycerol molecules. A sorbitan fatty acid ester molecule consists on mono-, di- or polyesters of fatty acids with sorbitol, sorbitan and sorbide. A sucrose fatty acid ester molecule consists of mono-, di- or polyesters of fatty acids with sucrose. As with the acyl group of monoglyceride, the fatty acids/acyl groups of polyglycerol fatty acid ester, sorbitan fatty acid ester and sucrose fatty acid ester preferably have carbon chains of 8-22 carbon atoms and 1-4 unsaturations. As above, the specific acyl groups, purity, and mixture of agent molecules useful in the invention depend on the requirements of the individual user.

Any single agent or mixture of different agents which enhances hydrolysis of phosphatidylcholine is contemplated as useful for the invention. The aforementioned agents are available commercially from a variety of sources.

Phosphatidylcholine is hydrolyzed to lysophosphatidylcholine by the action of phospholipase A₂, which severs the ester bond linking a fatty acid group to the 2-position of the glycerol in the head group of phosphatidylcholine. Phospholipase A₂ may be purified from a variety of sources, or it may be obtained from commercial sources (e.g. Lecitase™ 10 L, Novo Nordisk, Denmark). For full activity, phospholipase A₂ is believed to require the presence of Ca²⁺ ions in the reaction mixture. While typically there is a low level of Ca²⁺ ions in the commercial phospholipase A₂ preparations such that phospholipase A₂ is active, it is preferred that Ca²⁺ ions be added to the reaction mixture for full activity. It should be noted that Ca²⁺ ions are depleted from the reaction mixture by ionic bonding with the acid group of fatty acids liberated during hydrolysis of phosphatidylcholine. Therefore it is preferred that sufficient Ca²⁻ ions are added to the reaction mixture to maintain full activity of phospholipase A₂. In this invention, it is most preferable that the user supplement the calcium ion concentration to achieve a molar ratio of calcium ion:phosphatidylcholine of at least 1:1.

It will be recognized by persons of skill in the art that other ions may be substituted for the Ca²⁺ ions in order to maintain full activity of the phospholipase A₂ enzyme. While not all ions may substitute for Ca²⁺ ions in this reaction, the specific type and concentration of ions adequate for maintenance of phospholipase A₂ activity easily may be tested by one of ordinary skill in the art.

As disclosed above, the method of making lysophosphatidylcholine comprises contacting an aqueous dispersion of the mixture of phosphatidyicholine and an agent with phospholipase A₂. It is believed that the aqueous nature of the reaction mixture facilitates the orientation of the polar portions of the phosphatidylcholine molecules such that a lamellar structure is formed. A lamellar structure is believed preferred for efficient hydrolysis of phosphatidylcholine to lysophosphatidylcholine by phospholipase A₂. It therefore is contemplated that nonaqueous mixtures of phosphatidylcholine and an agent could also be utilized in the method of the invention, if such nonaqueous mixtures correctly orient the phosphatidylcholine and agent molecules in a lamellar structure, and if the specificity of phospholipase A₂ hydrolysis is retained.

Other reaction conditions, such as pH, time and temperature, may be varied to achieve optimal hydrolysis of phosphatidylcholine. For example, phospholipase A₂ has a pH optimum of pH 8-9 which should be maintained to retain maximal enzyme activity. During the progress of the reaction, as fatty acids are released by hydrolysis of phosphatidylcholine, the pH of the reaction mixture may change. Such a change of pH may require the addition of base to maintain the optimal range of pH 8-9. Any base which effectively raises the pH to the optimal range without interfering with the hydrolysis of phosphatidylcholine may be used. Applicant has successfully used aqueous sodium hydroxide for this purpose, however other formulations of sodium hydroxide or other bases may be employed for the same purpose. If the particular reaction conditions employed result in an increase in pH, then it is contemplated that acid may be added to maintain optimal pH.

Hydrolysis of phosphatidylcholine by phospholipase A₂ will proceed at many temperatures, but it is preferred that the reaction is carried out at the optimal temperature for enzymatic activity (70-80°C). Such a temperature is preferred for the reason that the phosphatidylcholine/agent bilayer structure will be fluid yet coherent, allowing for access of the phospholipase A₂ to the phosphatidylcholine molecules. Other optimal temperatures may be determined with minimal experimentation by one of ordinary skill in the art depending on the specific reaction mixture employed in the method of the invention.

The time for the reaction may be chosen by the user of the method as is convenient, so long as the hydrolysis of phosphatidylcholine has progressed to an extent desired. It is preferred that the reaction proceed for 1 to 5 days, most preferably for 2 days.

As mentioned above, hydrolysis of phosphatidylcholine is believed most efficient when a lamellar structure is maintained in the reaction mixture. Hence it is preferred that the reaction components be combined in amounts which maintain a lamellar structure throughout the course of the reaction. The amount of phosphatidylcholine to be used in the method of the invention is quantified as a weight percentage of the total reaction mixture. Weight percentage is calculated by dividing the weight of a single reaction component divided by the sum of the weights of all reaction components.

It is preferred that the amount of phosphatidylcholine in the reaction mixture represent not more than about 40% by weight of the total reaction mixture. Reaction mixtures comprising more than about 40% phosphatidylcholine by weight are likely to separate into a non-lamellar two-phase system which does not permit efficient hydrolysis of the phosphatidylcholine. Most preferably, the phosphatidylcholine represents about 30% by weight of the total reaction mixture.

An agent may be added to the mixture at any weight percentage which enhances the hydrolysis of phosphatidylcholine over the amount of hydrolysis of phosphatidylcholine alone by phospholipase A₂. Most preferably, the agent is monoglyceride. When present in the reaction mixture, virtually any amount of monoglyceride will enhance the hydrolysis of phosphatidylcholine by phospholipase A₂. Preferably the molar ratio of phosphatidylcholine: monoglyceride is 1:0.1-1:10. To reach high yields of lysophosphatidylcholine it is preferred to have a molar ratio of phosphatidylcholine:monoglyceride of about 1:1-1:5. Most preferably, the molar ratio of phosphatidylcholine:monoglyceride is about 1:3.

The desired end products of the reaction of phosphatidylcholine and agent with phospholipase A₂ are lysophosphatidylcholine alone, a combination of lysophosphatidylcholine with fatty acid or agent, or lysophosphatidylcholine in combination with fatty acid and agent. In particular, when the agent is monoglyceride, a preferred end product is a lipid matrix comprising phosphatidylcholine, monoglyceride and fatty acid. The utility of this lipid matrix has been disclosed in U.S. patents 4,874,795 and 5,314,921.

Where the end product is a lipid matrix composition of lysophosphatidylcholine, monoglyceride and fatty acid, it is preferred that the constituents of the lipid matrix be present in the molar ratio of lysophosphatidylcholine:the sum of monoglyceride and fatty acid of about 1:3 to 1:12. Most preferably, the molar ratio of lysophosphatidylcholine:the sum of monoglyceride and fatty acid is about 1:5-1:6. It is also preferred that the individual components of the lipid matrix are present in particular molar ratios in relation to one another. Thus, it is preferred that the molar ratios of lysophosphatidylcholine:monoglyceride:fatty acid are 1:4:2-1:2:4. Most preferably, the molar ratios of lysophosphatidylcholine:monoglyceride:fatty acid are either 1:4:2, 1:3:3 or 1:3:2.

Additional monoglycerides and fatty acids may be added to the lysophosphatidylcholine/monoglyceride/fatty acid mixture and melted or mixed to yield compositions of matter as defined in U.S. patent no. 4,874,795. Thus, monoglyceride and/or fatty acid may be added to the lipid matrix if it is desired to alter the molar ratios of monoglyceride and/or fatty acid to yield a desired product.

The lipid matrix produced by the method of the invention is useful for, *inter alia,* delivery of drugs. When so desired, a pharmaceutical composition may be added to the reaction mixture, for inclusion in the lipid matrix, at any time which does not adversely affect the integrity of the pharmaceutical composition. Preferably the desired pharmaceutical composition is added subsequent to the formation of the lipid matrix.

Preferably the methods disclosed herein include a step of recovering from the reaction mixture the lysophosphatidylcholine formed in the reaction mixture. As used herein, "recovering" means recovering the lysophosphatidylcholine from one or more of the components of the reaction mixture. The actual form of the lysophosphatidylcholine can vary, i.e., the lysophosphatidylcholine recovered can be recovered complexed with other components of the reaction mixture. For example, recovering lysophosphatidylcholine includes recovering a lipid complex which contains lysophosphatidylcholine, fatty acid and agent. Recovering lysophosphatidylcholine also embraces recovering lipid complexes which contain lysophosphatidylcholine and agent or lysophosphatidylcholine and fatty acid. It is not necessary that the lysophosphatidylcholine or lysophosphatidylcholine-containing lipid complex be purified to be considered recovered. Therefore, the lysophosphatidylcholine or lysophosphatidylcholine-containing lipid complex can contain other constituents present in the reaction mixture, such as Ca²⁺ or phospholipase A₂. The lysophosphatidylcholine, however, when "recovered" is sufficiently isolated from other materials so as to be useful as an isolate of lysophosphatidylcholine or of a lysophosphatidylcholine-containing lipid complex. The lysophosphatidylcholine or lysophosphatidylcholine-containing lipid complex which is recovered can, however, be purified if so desired.

The step of recovering can include one or more process steps whereby lysophosphatidylcholine is separated from one or more of the constituents of the reaction mixture. Thus, lysophosphatidylcholine may be separated from fatty acid, agent (e.g. monoglyceride) or fatty acid and agent. Separation includes separation of the desired lysophosphatidylcholine or lysophosphatidylcholine-containing lipid complex from the reaction mixture as well as separation of an unwanted reaction component from the reaction mixture. For example, the reaction mixture can be extracted with acetone to preferentially separate lysophosphatidylcholine from other reaction mixture components, as described herein. In other embodiments, where a lipid matrix comprising lysophosphatidylcholine, monoglyceride and fatty acid is the desired end product, other reaction components such as phospholipase A₂, water, organic solvents and excesses of monoglyceride or fatty acid can be separated from the lipid matrix. Alternatively, water can be separated from other reaction mixture components by heating or drying the reaction mixture as is described herein. Other methods of separating selected products of the enzymatic hydrolysis of phosphatidylcholine are provided herein, and still others will be known to one of ordinary skill in the art.

Many methods known to those of ordinary skill in the art will be applicable to separation of lysophosphatidylcholine from other reaction components based on differential solubilities, molecular weights, molecular sizes or other properties. For example, lysophosphatidylcholine may be separated from other components by preparative silica gel chromatography. Preferably, lysophosphatidylcholine can be separated by extraction of the reaction mixture with acetone. This method relies on the insolubility of phospholipids in acetone; lysophosphatidylcholine precipitates as a solid which is easily recovered from other reaction constituents. Other separation methods will be known to those of ordinary skill in the art.

Compositions containing lysophosphatidylcholine, alone or in combination with monoglyceride and/or fatty acids, are useful as emulsifiers, antioxidants and surfactants in cosmetic and dermatological preparations.

As disclosed above, the methods of the invention also contemplate the removal of water and/or other solvents from the reaction mixture to recover desired end products. Thus, the method of making any of the foregoing products may include the removal of water or solvents as part of, or separate from, the separation processes outline above.

Any method known in the art for the removal of water, aqueous solvents, or mixtures of aqueous and organic solvents may be used so long as the desired end products of the hydrolysis reaction are not adversely affected. It is preferred that methods which are scalable to industrial production of lysophosphatidylcholine or lipid matrix compositions be employed. For example, solvents may be removed from desired end products by heating, lyophilization, or spray drying processes. Such methods may be employed for such a time and to such an extent so as to remove all or part of the water or solvent mixtures as desired by the user. Preferably, reaction products are heated to remove water, thereby yielding a paste of lysophosphatidylcholine or lipid matrix.

### Examples

### Example 1

Phospholipase A₂ was prepared from *Naja naja siamenesis* and *Crotalus atrox.* A stock solution containing 2 mg/ml of each enzyme was prepared in 10 mM borate buffer pH7.4 containing 0.74 mM Ca⁺². Lipid mixtures in hexane:ethanol (95/5) were prepared which contained the soy phosphatidylcholine (Phospholipon, Nattermann Phospholipid GMBH, lot # 60020; 250 µg/ml), 1-palmitoyl-2-(1-[¹⁴C-oleoyl])-phosphatidylcholine (~800,000 dpm, negligible mass) and monoolein [MO] (4.54 mg/ml). The molar ratios of phosphatidylcholine [PC] to MO in the 6 samples were 1:0, 1:1, 1:2, 1:3, 1:4 and 1:5. Each sample in a 1 ml "ReactiVial" (Pierce, Rockford, IL) was taken to dryness under N₂. Borate/Ca⁺² buffer, 0.01 M pH 7.4, was added. the sample was vortexed and then sonicated for 10 min. at 47°C in a Branson sonifier (Model W-375) equipped with a cuphorn at a power level of 6-7. The reaction mixture contained 250 µg PC and 4 µg of each phospholipase A₂ preparation in a total volume of 0.2 ml buffer. Monoolein was present in the samples at the molar ratios indicated above. The samples were incubated at 30°C in a shaker bath with aliquots being withdrawn for analysis.

After the analysis of radioactivity recovered from 10 µl aliquots withdrawn at 5hr. it was apparent that the lipids in all but the 1:0 and 1:1 were not homogeneously distributed, *i.e*., that we were not obtaining a representative sample of the vial contents. This was also obvious from inspection of the samples which showed aggregated lipid adhering to the walls of the 1:≥2 samples. All vials were resonicated in the cuphorn sonicator and assayed again at 24 hr. With the exception of the 1:2 sample the appearance of samples 1:≥3 was unchanged and recovered radioactivity indicated that these samples were still not uniformly dispersed. At approximately 24 hr. following addition of enzyme to the vials, 100 µl of buffer was added to each and each sample, heated to 50°C was sonicated for 30 sec. using a Branson Sonifier (Model W225) at a power level of 2-3 using a microtip placed directly into the sample. This dispersed the lipid in all samples, although coagulation and poor recovery radioactivity was observed subsequently in the 1:4 and 1:5 samples. Immediately following this direct sonication, another aliquot of enzyme solution was added to each sample and incubation at 30°C was resumed. Aliquot size withdrawn for analysis was increased to 20 µl. After an elapsed time of 240 hr. following addition of enzyme, all remaining sample was extracted and analyzed in the same manner as the aliquots withdrawn at intermediate time points.

Aliquots withdrawn for analysis were added to 0.6 ml of CHCl₃:methanol (2:1) and 0.3 ml of 2mM EGTA pH 5.25 in water. The organic solvent contained PC at 0.0417 mg/ml, lysophosphatidylcholine at 0.0417 mg/ml, oleic acid [OA] at 0.0417 mg/ml and MO at 0.0417 mg/ml to give final carrier lipid amounts of 25 µg of each species per sample to be subjected to thin layer chromatography. The samples were vortexed and centrifuged in a Sorvall SS-34 rotor at 3000 x g for 10 minutes at room temperature to separate organic and aqueous phases. The lower organic phase was removed from the vial with a syringe and placed in a small test tube and the samples were dried under N₂ (the aqueous phase dried down and was checked for radioactivity in a scintillation counter and there was <1200 dpm out of a possible 800,000 dpm used). The samples were redissolved with 60 µl of 2:1 CHCl₃:methanol, spotted on alternate lanes under N₂ on a 20 x 20 Whatman LK5D (150 Å, 250 µm thickness) 19-channel scored silica gel TLC plate. The plate was developed in 60:40:1 hexane:diethyl ether:acetic acid. The solvent was evaporated in air and the plate was scored 7 cm from the bottom. A second development in 60:40:1 CHCl₃:methanol:NH₄OH was used to move the PC off the origin and move the MO away from the PC. The radiolabeled product oleic acid is well above the 7 cm front. The plate was dried and a the distribution of radioactivity in each lane was determined using a Berthode Linear Analyser radioactivity detector. This unit gives an apparent efficiency of approximately 7-9%. In a sham experiment enzyme was added to the extraction mixture immediately after the aliquot of incubation sample. No hydrolysis of the radiolabeled PC occurred, indicating that the conditions in the extraction medium precluded the expression of phospholipase activity during sample workup.

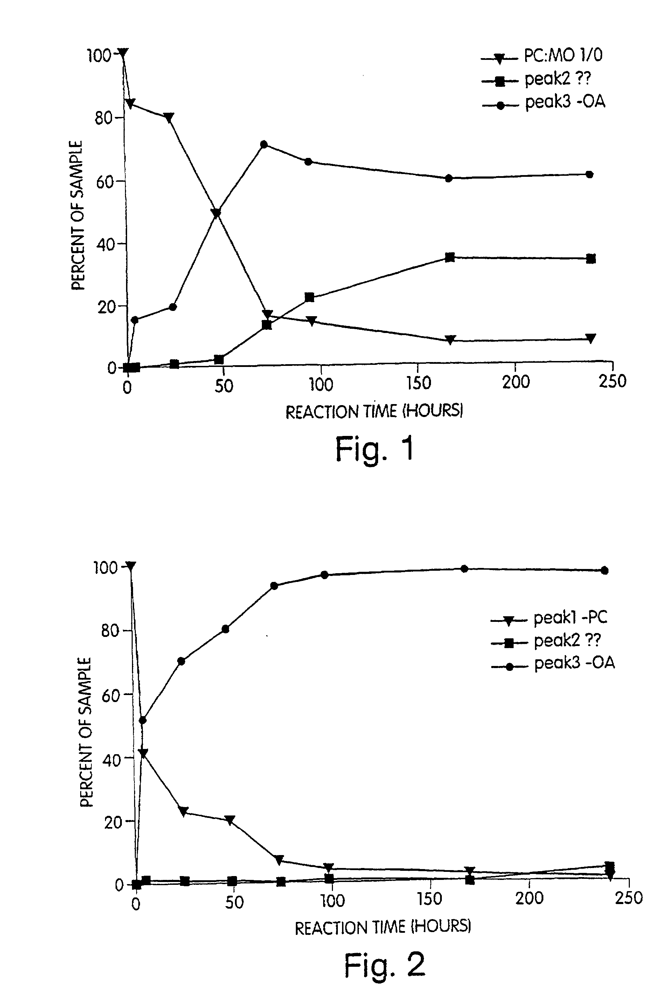

The results of the experiments are tabulated in Table 1 below and graphically represented in Figs. 1 and 2. The results are consistent with a maximal conversion of phosphatidylcholine to lysophosphatidylcholine of about 70% without added monoglyceride (ratio of PC:MO = 1:0), as measured by the appearance of radiolabeled oleic acid hydrolyzed from radiolabeled phosphatidylcholine. Addition of monoglyceride at any molar ratio increases the amount of lysophosphatidylcholine formed as measured by the appearance of radiolabeled oleic acid. It should also be noted that addition of monoglyceride to the reaction mixture reduces the accumulation of side product, virtually eliminating side product production at all molar ratios of PC:MO at times up to and including 96 hours. Even at longer reaction times, side product production is reduced. The side product is believed to be glycerophosphatidylcholine. Studies to confirm the increase in lysophosphatidylcholine formation in the presence of monoglyceride are presently performed using phosphatidylcholine having a labeled ¹⁴C-choline group.

**Table 1:**

| Effect of Monoolein addition on the PLA₂ Enzymatic Hydrolysis of Phosphatidylcholine | | | | | | |
|---|---|---|---|---|---|---|
| | Molar Ratio of PC:MO | | | | | |
| Time(hr.) | 1:0 | 1:1 | 1:2 | 1:3 | 1:4 | 1:5 |
| PC | | | | | | |
| 0 | 100 | 100 | 100 | 100 | 100 | 100 |
| 5 | 84 | 67 | 49 | 41 | 14 | 57 |
| 24 | 79 | 41 | 29 | 22 | 18 | 31 |
| 48 | 49 | 16 | 26 | 19 | 27 | 14 |
| 72 | 15 | 9 | 9 | 6 | 13 | 4 |
| 96 | 13 | 11 | 6 | 3 | 9 | 3 |
| 168 | 7 | 6 | 3 | 2 | * | * |
| 240 | 7 | 5 | 5 | 1 | 1 | 1 |

| OA | | | | | | |
|---|---|---|---|---|---|---|
| 0 | ND | ND | ND | ND | ND | ND |
| 5 | 15 | 32 | 46 | 51 | 64 | 35 |
| 24 | 19 | 58 | 65 | 70 | 66 | 59 |
| 48 | 49 | 82 | 73 | 80 | 71 | 83 |
| 72 | 70 | 89 | 89 | 93 | 84 | 93 |
| 96 | 64 | 86 | 92 | 95 | 83 | 93 |
| 168 | 59 | 71 | 87 | 97 | * | * |
| 240 | 59 | 64 | 75 | 96 | 86 | 95 |

| side product | | | | | | |
|---|---|---|---|---|---|---|
| 0 | ND | ND | ND | ND | ND | ND |
| 5 | 0 | 0 | 0 | 1 | 1 | 0 |
| 24 | 0 | 0 | 0 | 1 | 3 | 1 |
| 48 | 2 | 0 | 0 | 1 | 1 | 1 |
| 72 | 12 | 1 | 1 | 0 | 1 | 1 |
| 96 | 21 | 2 | 2 | 1 | 3 | 1 |
| 168 | 33 | 22 | 9 | 0 | * | * |
| 240 | 32 | 28 | 20 | 3 | 12 | 3 |

| | | | | | | |
|---|---|---|---|---|---|---|
| * insufficient radioactivity in aliquots for quantitative analysis; ND not determined. | | | | | | |

### Example 2

Phosphatidylcholine (Phospholipon® 80, estimated molecule weight 785, American Lecithin, contains approximately 80% phosphatidylcholine) and monoglycerides (Dimodan™ LSK and Dimodan™ OK, estimated molecular weight 356, Danisco Ingredients) are combined at a phosphatidylcholine:monoglyceride molar ratio of 1:1 to 1:5. The preferred ratio of phosphatidylcholine:monoglyceride is 1:3. These components are mixed together and heated at 70-80°C to obtain a uniform melt, i.e., absence of any observable schleiren. Sufficient water is then added so that phosphatidylcholine represents 30% by weight of the total reaction components. The reaction components are mixed and heated at the reaction temperature of 70-80°C to obtain a uniform consistency. The pH is adjusted and maintained at approximately pH 8 to pH 9 with aqueous sodium hydroxide. Lecitase™ 10L, a phospholipase A₂ enzyme (Novo Nordisk. Denmark), is added at approximately 2 ml per kg of Phospholipon® 80. Further addition of calcium ions is not necessary, presumably due to the calcium ions present in the enzyme preparation. The reaction conditions of temperature, stirring and pH are maintained for 2 days, until the hydrolysis of phosphatidylcholine is complete. After hydrolysis is complete, the water is removed with heat to yield a paste.

Additional monoglyceride and fatty acid optionally is added to the lysophosphatidylcholine/monoglyceride/fatty acid lipid matrix and melted together with mixing according to the above method to yield compositions of matter as defined in U.S. patent no. 4,874,975.

To obtain high purity lysophosphatidylcholine, the end products of the reaction are precipitated by acetone, which extracts both the monoglycerides and the fatty acids from the lysophosphatidylcholine/monoglyceride/fatty acid hydrolysis products described above. Upon addition of acetone, lysophosphatidylcholine precipitates out of solution, and may be recovered by any art-standard process.

## Claims

1. A method for making lysophosphatidylcholine comprising:
combining an agent and phosphatidylcholine with water to form in the water an aqueous dispersion of a mixture of the agent and phosphatidylcholine, and
contacting the aqueous dispersion of the mixture with phospholipase A₂ to form a reaction mixture, wherein the agent is selected from the group consisting of monoglyceride, diglyceride, polyglycerol fatty acid ester, sucrose fatty acid ester, sorbitan fatty acid ester, and glycerol.

2. The method of claim 1, further comprising recovering lysophosphatidylcholine formed in the reaction mixture.

3. The method of claim 2, wherein the step of recovering comprises separating lysophosphatidylcholine from a reaction mixture constituent selected from the group consisting of fatty acid, the agent, or fatty acid and the agent.

4. The method of claim 3, wherein separation of lysophosphatidylcholine from fatty acid and the agent comprises extraction with acetone.

5. The method of any of the preceding claims, wherein the agent is monoglyceride.

6. The method of claim 1, wherein the agent is monoglyceride having an acyl group and the acyl group has between 8 and 22 carbon atoms.

7. The method of claim 1, wherein the aqueous dispersion of the mixture of an agent and phosphatidylcholine is contacted with phospholipase A₂ in the presence of calcium ions.

8. The method of claim 1, wherein the phosphatidylcholine in the mixture is less than about 40% by weight of the mixture, preferably about 30% by weight of the mixture.

9. A method for making lysophosphatidylcholine comprising:
combining an agent, phosphatidylcholine, and an organic solvent with water to form in the water an aqueous dispersion, containing the organic solvent, of a mixture of the agent, phosphatidylcholine, wherein the solvent is selected from the group consisting of diethyl ether, tertiary butyl alcohol, diethyl ether/ethanol mixtures, tertiary butyl alcohol/ethanol mixtures, and methyl isobutyl ketone, and
contacting the aqueous dispersion of the mixture with phospholipase A₂ to form a reaction mixture, wherein the agent is selected from the group consisting of monoglyceride, diglyceride, polyglycerol fatty acid ester, sucrose fatty acid ester, sorbitan fatty acid ester, and glycerol.

10. A method for making lysophosphatidylcholine comprising:
combining phosphatidylcholine and monoglyceride with water to form in the water an aqueous dispersion of a mixture of phosphatidylcholine and monoglyceride, wherein the ratio of phosphatidylcholine:monoglyceride is from about 1:1 to about 1:5, preferably about 1:3, and
contacting the aqueous dispersion of the mixture with phospholipase A₂ to form a reaction mixture.

11. A method for making a composition containing lysophosphatidylcholine, monoglyceride and fatty acid, comprising:
contacting an aqueous mixture of phosphatidylcholine and monoglyceride with phospholipase A₂, and
recovering a lipid complex containing lysophosphatidylcholine, monoglyceride and fatty acid.

12. The method of claim 11, wherein the step of recovering the lipid complex comprises removal of water.

13. The method of claim 11, wherein the molar ratio of lysophosphatidylcholine:the sum of monoglyceride and fatty acid in the recovered lipid complex composition is between 1:3 and 1:12, preferably between 1:5 and 1:6.

14. The method of claim 13, wherein the recovered lipid complex composition has a lysophosphatidylcholine:monoglyceride:fatty acid molar ratio between 1:4:2 and 1:2:4, preferably selected from the group consisting of 1:4:2, 1:3:3 and 1:3:2.

15. The method of claim 11 or 14, wherein the monoglyceride is derived from natural triglyceride.

16. A method for making lysophosphatidylcholine comprising:
combining an agent and phosphatidylcholine with water to form in the water an aqueous dispersion of a mixture of the agent and phosphatidylcholine, and
contacting the aqueous dispersion of the mixture with phospholipase A₂ to form a reaction mixture, wherein the agent is selected from the group consisting of monoglyceride, diglyceride, polyglycerol fatty acid ester, sucrose fatty acid ester, sorbitan fatty acid ester, and glycerol, and wherein the agent is present in an amount effective to enhance the conversion of phosphatidylcholine to lysophosphatidylcholine in the reaction mixture compared to the conversion of phosphatidylcholine to lysophosphatidylcholine in the reaction mixture absent the agent.

17. The method of claim 16, wherein the agent is present in an amount effective to increase to 80% or greater, preferably 90%, 95% or 99%, the efficiency of conversion of phosphatidylcholine to lysophosphatidylcholine in the reaction mixture.

18. A method for making lysophosphatidylcholine comprising:
combining an agent and phosphatidylcholine to form a mixture of the agent and phosphatidylcholine, wherein the agent is selected from the group consisting of monoglyceride, diglyceride, polyglycerol fatty acid ester, sucrose fatty acid ester, sorbitan fatty acid ester, and glycerol,
combining the mixture with water to form in the water an aqueous dispersion of the mixture, and
contacting the aqueous dispersion of the mixture with phospholipase A₂ to form a reaction mixture.

## Patentansprüche

1. Ein Verfahren zum Herstellen von Lysophosphatidylcholin umfassend:
Vereinigen eines Agens und Phosphatidylcholin mit Wasser, um in dem Wasser eine wässrige Dispersion einer Mischung des Agens und Phosphatidylcholin zu bilden, und
Kontaktieren der wässrigen Dispersion der Mischung mit Phospholipase A₂, um eine Reaktionsmischung zu bilden, wobei das Agens ausgewählt ist aus der Gruppe umfassend Monoglyceride, Diglyceride, Polyglycerolfettsäureester, Saccharosefettsäureester, Sorbitanfettsäureester und Glycerin.

2. Das Verfahren nach Anspruch 1, weiter umfassend Aufarbeiten des in der Reaktionsmischung gebildeten Lysophosphatidylcholins.

3. Das Verfahren nach Anspruch 2, wobei der Schritt des Aufarbeitens Abtrennen von Lysophosphatidylcholin von einem Reaktionsmischungsbestandteil umfasst, der ausgewählt ist aus der Gruppe, die Fettsäure, das Agens, oder Fettsäure und das Agens umfasst.

4. Das Verfahren nach Anspruch 3, wobei das Abtrennen von Lysophosphatidylcholin von Fettsäure und dem Agens Extraktion mittels Aceton umfasst.

5. Das Verfahren nach einem des vorangehenden Ansprüche, wobei das Agens Monoglycerid ist.

6. Das Verfahren nach Anspruch 1, wobei das Agens Monoglycerid mit einer Acylgruppe ist und die Acylgruppe zwischen 8 und 22 Kohlenstoffatomen aufweist.

7. Das Verfahren nach Anspruch 1, wobei die wässrige Dispersion der Mischung aus einem Agens und Phosphatidylcholin mit Phospholipase A₂ in Gegenwart von Calciumionen kontaktiert wird.

8. Das Verfahren nach Anspruch 1, wobei das Phosphatidylcholin in der Mischung weniger als etwa 40 Gewichtsprozent der Mischung, bevorzugter Weise etwa 30 Gewichtsprozent der Mischung ist.

9. Ein Verfahren zum Herstellen von Lysophosphatidylcholin umfassend:
Vereinigen eines Agens, Phosphatidylcholin und eines organischen Lösungsmittels mit Wasser, um in dem Wasser eine das organische Lösungsmittel enthaltende wässrige Dispersion aus einer Mischung des Agens und Phosphatidylcholin zu bilden, wobei das Lösungsmittel ausgewählt ist aus der Gruppe, die Diethylether, tertiären Butylalkohol, Diethylether/Ethanol-Mischungen, Mischungen aus tertiärem Butylalkohol und Ethanol, und Methylisobutylketon umfasst, und
Kontaktieren der wässrigen Dispersion der Mischung mit Phospholipase A₂, um eine Reaktionsmischung zu bilden, wobei das Agens ausgewählt ist aus der Gruppe, die Monoglyceride, Diglyceride, Polyglycerolfettsäureester, Saccharosefettsäureester, Sorbitanfettsäureester und Glycerin umfasst.

10. Ein Verfahren zum Herstellen von Lysophosphatidylcholin umfassend:
Vereinigen von Phosphatidylcholin und Monoglycerid mit Wasser, um in dem Wasser eine wässrige Dispersion aus einer Mischung von Phosphatidylcholin und Monoglycerid zu bilden, wobei das Verhältnis von Phosphatidylcholin zu Monoglycerid von etwa 1:1 bis etwa 1:5, bevorzugter Weise 1:3 beträgt, und
Kontaktieren der wässrigen Dispersion der Mischung mit Phospholipase A₂, um eine Reaktionsmischung zu bilden.

11. Ein Verfahren zum Herstellen einer Lysophosphatidylcholin, Monoglycerid und Fettsäure umfassenden Zusammensetzung, umfassend:
Kontaktieren einer wässrigen Mischung aus Phosphatidylcholin und Monoglycerid mit Phospholipase A₂, und
Aufarbeiten eines Lipidkomplexes, der Lysophosphatidylcholin, Monoglycerid und Fettsäure enthält.

12. Das Verfahren nach Anspruch 11, wobei der Schritt des Aufarbeitens des Lipidkomplexes Entfernen von Wasser umfasst.

13. Das Verfahren nach Anspruch 11, wobei das molare Verhältnis von Lysophosphatidylcholin:Summe aus Monoglycerid und Fettsäure in der aufgearbeiteten Lipidkomplex-Zusammensetzung zwischen 1:3 und 1:12, bevorzugter Weise zwischen 1:5 und 1:6 beträgt.

14. Das Verfahren nach Anspruch 13, wobei die aufgearbeitete Lipidkomplex-Zusammensetzung ein molares Verhältnis von Lysophosphatidylcholin:Monoglycerid:Fettsäure zwischen 1:4:2 und 1:2:4 aufweist, bevorzugter Weise ausgewählt ist aus der Gruppe umfassend 1:4:2, 1:3:3 und 1:3:2.

15. Das Verfahren nach Anspruch 11 oder 14, wobei das Monoglycerid von natürlichem Triglycerid gewonnen ist.

16. Ein Verfahren zum Herstellen von Lysophosphatidylcholin umfassend:
Vereinigen eines Agens und Phosphatidylcholin mit Wasser, um in dem Wasser eine wässrige Dispersion einer Mischung aus dem Agens und Phosphatidylcholin zu bilden, und
Kontaktieren der wässrigen Dispersion der Mischung mit Phospholipase A₂, um eine Reaktionsmischung zu bilden, wobei das Agens ausgewählt ist aus der Gruppe, die Monoglycerid, Diglycerid, Polyglycerolfettsäureester, Saccharosefettsäureester, Sorbitanfettsäureester und Glycerol umfasst, und wobei das Agens in einer wirksamen Menge vorhanden ist, um die Umwandlung von Phosphatidylcholin in Lysophosphatidylcholin in der Reaktionsmischung zu verstärken verglichen mit der Umwandlung von Phosphatidylcholin zu Lysophosphatidylcholin in der Reaktionsmischung ohne das Agens.

17. Das Verfahren nach Anspruch 16, wobei das Agens in einer wirksamen Menge vorhanden ist, um die Umwandlungseffizienz von Phosphatidylcholin in Lysophosphatidylcholin in der Reaktionsmischung auf 80% oder höher, bevorzugter Weise 90%, 95% oder 99% zu erhöhen.

18. Ein Verfahren zur Herstellung von Lysophosphatidylcholin umfassend:
Vereinigen eines Agens und Phosphatidylcholin, um eine Mischung aus dem Agens und Phosphatidylcholin zu bilden, wobei das Agens ausgewählt ist aus der Gruppe, die Monoglycerid, Diglycerid, Polyglycerolfettsäureester, Saccharosefettsäureester, Sorbitanfettsäureester und Glycerol umfasst,
Vereinigen der Mischung mit Wasser, um in dem Wasser eine wässrige Dispersion der Mischung zu bilden, und
Kontaktieren der wässrigen Dispersion der Mischung mit Phospholipase A₂, um eine Reaktionsmischung zu bilden.

## Revendications

1. Une méthode pour la préparation de la lysophosphatidylcholine, cette méthode consistant à :
combiner un agent et de la phosphatidylcholine avec de l'eau pour former dans l'eau une dispersion aqueuse de l'agent et de la phosphatidylcholine, et
amener en contact la dispersion aqueuse du mélange avec de la phospholipase A₂ de manière à former un mélange réactionnel, l'agent étant choisi dans te groupe constitué par les monoglycérides, les diglycérides, les esters d'acides gras avec un polyglycérol, les esters d'acides gras avec le sucrose, les esters d'acides gras avec le sorbitane et le glycérol.

2. La méthode de la revendication 1, consistant au surplus à récupérer la lysophosphatidylcholine formée dans le mélange réactionnel.

3. La méthode de la revendication 2, dans laquelle l'étape de récupération consiste à séparer la lysophosphatidylcholine d'un constituant du mélange réactionnel choisi dans le groupe constitué par l'acide gras, l'agent, ou l'acide gras et l'agent.

4. La méthode de la revendication 3, dans laquelle la séparation de la lysophosphatidylcholine d'avec l'acide gras et l'agent consiste en une extraction par l'acétone.

5. La méthode selon l'une quelconque des revendications précédentes, dans laquelle l'agent est un monoglycéride.

6. La méthode de la revendication 1, dans laquelle l'agent est un monoglycéride présentant un groupe acyle, le groupe acyle comportant entre 8 et 22 atomes de carbone.

7. La méthode de la revendication 1, dans laquelle la dispersion aqueuse du mélange d'un agent et de la phosphatidylcholine est amenée en contact avec la phospholipase A₂ en présence d'ions calcium.

8. La méthode de la revendication 1, dans laquelle la phosphatidylcholine dans le mélange représente moins qu'environ 40% en poids du mélange et de préférence environ 30% en poids du mélange.

9. Une méthode pour la préparation de la lysophosphatidylcholine consistant à:
combiner un agent, de la phosphatidylcholine et un solvant organique avec de l'eau de manière à former, dans l'eau, une dispersion aqueuse contenant le solvant organique, un mélange de l'agent et de la phosphatidylcholine, le solvant étant choisi dans le groupe constituant par l'éther diéthylique, l'alcool tertiobutylique, les mélanges éther diéthylique/éthanol, les mélanges alcool tertiobutylique/éthanol et la méthylisobutylcétone, et
amener en contact la dispersion aqueuse du mélange avec de la phospholipase A₂ de manière à former un mélange réactionnel, l'agent étant choisi dans le groupe constitué par les monoglycérides, les diglycérides, les esters d'acides gras avec un polyglycérol, les esters d'acides gras avec le sucrose, les esters d'acides gras avec le sucrose, les esters d'acides gras avec le sorbitane et le glycérol.

10. Une méthode pour la préparation de la lysophosphatidylcholine consistant à:
combiner de la phosphatidylcholine et un monoglycéride avec de l'eau de manière à former, dans l'eau, une dispersion aqueuse d'un mélange de phosphatidylcholine et de monoglycéride, dans lequel la proportion phosphatidylcholine : monoglycéride est d'environ 1:1 à environ 1:5 et de préférence d'environ 1:3 et
amener en contact la dispersion aqueuse du mélange avec de la phospholipase A₂ de manière à former un mélange réactionnel.

11. Une méthode pour la préparation d'une composition contenant de la lysophosphatidylcholine, un monoglycéride et un acide gras, méthode consistant à :
amener en contact un mélange aqueux de phosphatidylcholine et d'un monoglycéride avec de la phospholipase A₂ et
récupérer un lipide complexe contenant de la lysophosphatidylcholine, un monoglycéride et un acide gras.

12. La méthode de la revendication 11, dans laquelle l'étape de récupération du lipide complexe consiste en l'élimination de l'eau.

13. La méthode de la revendication 11, dans laquelle le rapport molaire lysophosphatidylcholine : somme du monoglycéride et de l'acide gras dans le lipide complexe récupéré est compris entre 1:3 et 1:12 et de préférence entre 1:5 et 1:6.

14. La méthode de la revendication 13, dans laquelle le lipide complexe récupéré présente un rapport molaire lysophosphatidylcholine : monoglycéride : acide gras compris entre 1:4:2 et 1:2:4 et de préférence choisi dans le groups constitué par 1:4:2, 1:3:3 et 1:3:2.

15. La méthode de la revendication 11 ou 14, dans laquelle le monoglycéride est dérivé d'un triglycéride naturel.

16. Une méthode pour la préparation de la lysophosphatidylcholine consistant à:
combiner un agent et de la phosphatidylcholine avec de l'eau pour former, dans l'eau, une dispersion aqueuse d'un mélange de l'agent et de la phosphatidylcholine, et
amener en contact la dispersion aqueuse du mélange avec de la phospholipase A₂ de manière à former un mélange réactionnel, l'agent étant choisi dans le groupe constitué par les monoglycérides, les diglycérides, les esters d'acides gras avec un polyglycérol, les esters d'acides gras avec le sucrose, les esters d'acides gras avec le sorbitane et le glycérol, et l'agent étant présent en une quantité suffisante pour augmenter la conversion de la phosphatidylcholine en lysophosphatidylcholine dans le mélange réactionnel, par rapport à la conversion de la phosphatidylcholine en lysophosphatidylcholine dans le mélange réactionnel dont est absent l'agent.

17. La méthode de la revendication 16, dans laquelle l'agent est présent en une quantité suffisante pour augmenter à 80% ou plus et de préférence à 90%, 95% ou 99% l'efficacité de la conversion de la phosphatidylcholine en lysophosphatidylcholine dans le mélange réactionnel.

18. Une méthode de préparation de la lysophosphatidylcholine consistant à :
combiner un agent et de la phosphatidylcholine de manière à former un mélange de l'agent et de la phosphatidylcholine, l'agent étant sélectionné dans le groupe constitué par les monoglycérides, les diglycérides, les esters d'acides gras avec un polyglycérol, les esters d'acides gras avec le sucrose, les esters d'acides gras avec le sorbitane et le glycérol,
combiner le mélange avec de l'eau de manière à former, dans l'eau, une dispersion aqueuse du mélange, et
amener en contact la dispersion aqueuse du mélange avec de la phospholipase A₂ de manière à former un mélange réactionnel.
